Europäisches Patentamt

European Patent Office

Office européen des brevets

(19)

(11) Veröffentlichungsnummer : **0 339 506 B1**

(12) **EUROPÄISCHE PATENTSCHRIFT**

(45) Veröffentlichungstag der Patentschrift :
**13.05.92 Patentblatt 92/20**

(51) Int. Cl.$^5$ : **A61K 31/135,** A61K 9/22,
A61K 9/24

(21) Anmeldenummer : **89107213.4**

(22) Anmeldetag : **21.04.89**

(54) Orales osmotisches System mit verbesserter Haftwirkung der Membran am Kern.

(30) Priorität : **25.04.88 US 185564**

(43) Veröffentlichungstag der Anmeldung :
**02.11.89 Patentblatt 89/44**

(45) Bekanntmachung des Hinweises auf die
Patenterteilung :
**13.05.92 Patentblatt 92/20**

(84) Benannte Vertragsstaaten :
**AT BE CH DE ES FR GB GR IT LI LU NL SE**

(56) Entgegenhaltungen :
EP-A- 0 153 105
EP-A- 0 220 143
EP-A- 0 271 438
US-A- 3 845 770
US-A- 4 609 374
US-A- 4 693 886

(73) Patentinhaber : **CIBA-GEIGY AG
Klybeckstrasse 141
CH-4002 Basel (CH)**

(72) Erfinder : **Shah, Shailesh B., Dr.
416 Huntington Road
Union New Jersey 07204 (US)**
Erfinder : **Koparkar, Arun Dattatreya
1005 Columbus Avenue
Westfield New Jersey 07090 (US)**

(74) Vertreter : **Zumstein, Fritz, Dr. et al
Bräuhausstrasse 4
W-8000 München 2 (DE)**

EP 0 339 506 B1

## Beschreibung

Orale osmotische Abgabesysteme sind bekannt. Diese therapeutischen Systeme in Form von beschichteten Tabletten geben den Wirkstoff auf kontrollierte und kontinuierliche Weise in einem verlängerten Zeitintervall ab und sind beispielsweise in den U.S. Patentschriften 3845770, 3,916,899 und 4,016,880 beschrieben.

Solche tablettenförmigen Systeme sind durch eine äussere semipermeable Membranwand gekennzeichnet, welche für Körperflüssigkeit im Gastrointestinaltrakt durchlässig ist, durch einen wirkstoffhaltigen Kern, welcher pharmazeutische Hilfsstoffe wie Bindemittel, osmotisch wirksame Treibmittel und weitere Tablettierhilfsstoffe enthalten kann, sowie durch mindestens eine Passage durch die äussere semipermeable Membran, durch die die Abgabe des Wirkstoffs erfolgt und welche einen Materialfluss zwischen wirkstoffhaltigem Kern und der äusseren Umgebung herstellt.

Nach der Verabreichung nimmt das therapeutische System im Gastrointestinaltrakt durch Einwirkung von Diffusion Körperflüssigkeit durch die semipermeable äussere Membran auf. Die Körperflüssigkeit löst anschliessend den Wirkstoff und/oder das lösliche osmotische Treibmittel auf, wodurch sich eine Lösung oder zumindest Suspension bildet, welche auf die äussere starre semipermeable Membran einen osmotischen Druck oder ein Druckgefälle ausübt. Da die äussere Membran starr und nur für Wasser aber nicht für Bestandteile der osmotisch aktiven Lösung oder Suspension permeabel ist, kann der osmotische Druck nur durch Abgabe aus dem Inneren des Systems ausgeglichen werden, so dass die osmotisch wirksamen Bestandteile der gebildeten Lösung oder Suspension mit dem Wirkstoff das therapeutische System durch die freie Passage in der äusseren Membran verlassen. Da sich nach Einnahme des therapeutischen Systems in Kontakt mit Körperflüssigkeit Gleichgewichtsvorgänge einstellen, erfolgt die Abgabe des Wirkstoffs kontrolliert, kontinuierlich und verzögert. Materialien, welche sich für die Herstellung der äusseren semipermeablen Membran eignen, sowie Funktionsweise und Herstellungsangaben für die oralen osmotischen Systeme sind in der U.S. Patentschrift 3,916,899 beschrieben.

Viele im Kern von solchen Systemen enthaltenen festen Wirkstoffe und osmotischen Treibmittel besitzen ungenügende Hafteigenschaften am filmartigen Material, welches die äussere semipermeable Membranwand bildet. Selbst beim gleichmässigen Beschichten eines solchen Kerns mit den filmartigen Ueberzugsmaterialien erhält man Systeme mit ungenügenden Hafteigenschaften des inneren Kerns an der äusseren semipermeablen Membranwand. Besonders gross ist die Gefahr, dass die äussere Membran vom Kern abblättert. Dieses Problem tritt bei den oft angewendeten Beschichtungsverfahren besonders gravierend auf, wenn geformte Tablettenkerne mit Lösungen besprüht werden, welche das Beschichtungsmittel für die äussere semipermeable Membranwand enthalten, z.B. bei der Anwendung der üblichen Luftwirbelverfahren, z.B. nach Wurster. Dabei kann sich der tablettenförmige Kern auch noch statisch aufladen und die einheitliche Haftwirkung zwischen Kern und Membran weiter herabsetzen.

Sogar bei Verwendung grösserer Mengen von üblichen polymeren Bindemitteln wie Poly-N-vinylpyrrolidon, Poly-$C_2$-$C_3$-alkylenglycolen oder Hydroxyalkylcelluloseäthern oder Mischungen davon und deren gleichförmiger Verteilung in dem den Tablettenkern bildenden Material wird eine schwache Haftwirkung zwischen dem Kern selbst und der äusseren Membranwand beobachtet, was sich durch Abblättern des Membranmaterials bei Kontakt mit Wasser oder Körperflüssigkeit äussert.

Die Verwendung grösserer Mengen polymerer Bindemittel ist dann ungünstig, wenn der Kern des therapeutischen Systems ausserdem grössere Wirkstoffmengen für eine Dosiseinheit aufnehmen muss. Dies erfordert dann die Herstellung eines voluminösen Systems, dessen Verabreichung schwierig ist. Ausserdem können grössere Mengen polymerer Bindemittel das gewünschte kontrollierte und kontinuierliche Abgabeprofil des Wirkstoffs durch zu grosse Abgabeverzögerung unterhalb des therapeutischen Niveaus nachteilig beeinträchtigen oder eine oder mehrere Passagen verstopfen und dadurch das Aufbrechen des Systems verursachen.

Der vorliegenden Erfindung liegt die Aufgabe zugrunde, ein verbessertes orales osmotisches Abgabesystem mit dem Wirkstoff Metoprolol herzustellen. Das Abgabesystem soll verbesserte Hafteigenschaften der äusseren semipermeablen Membranwand auf dem geformten inneren Tablettenkern besitzen und die Nachteile der bekannten oralen osmotischen Systeme wie Abblättern der äusseren Schicht und Aufbrechen unter Einwirkung von Körperflüssigkeit beseitigen.

Diese Aufgabenstellung wird durch die vorliegende Erfindung gelöst, welche ein orales osmotisches Abgabesystem mit dem Wirkstoff Metoprolol und verbesserten Stabilitätseigenschaften betrifft.

Es wurde überraschenderweise gefunden, dass bei einer zusätzlichen gleichmässigen Beschichtung des wirkstoffhaltigen Kerns mit einer separaten inneren dünnen und wasserlöslichen Filmschicht ein stabiles orales osmotisches System mit verbesserter Haftwirkung der äusseren Membran sich herstellen lässt.

Der innen beschichtete Kern lässt sich mit dem Material für die semipermeable äussere Membran beschichten, welche so mit dem inneren Kern fest verbunden bleibt.

Das erfindungsgemässe Abgabesystem eignet sich für die kontrollierte und kontinuierliche Applikation von Metoprolol in einem verlängerten Zeitintervall und gibt beispielsweise 50 bis 90 % der enthaltenen Wirkstoffmenge mit einer Abgabeleistung von 5-12 % stündlich an den Gastrointestinaltrakt ab.

Das erfindungsgemässe orale osmotische System enthält folgende Bestandteile:

a) einen geformten Tablettenkern mit einem osmotisch aktiven Gehalt eines pharmazeutisch annehmbaren Metoprololsalzes kombiniert mit einem pharmazeutisch annehmbaren und für Tablettierverfahren üblichen Bindemittel und gegebenenfalls einem osmotisch aktiven Treibmittel und/oder weiteren pharmazeutischen Hilfsstoffen;

b) eine gleichmässige, separate, innere Schicht aus ca. 0,3 % - 10 % (Gew.) wasserlöslichem Beschichtungsmittel bezogen auf das Gewicht des Tablettenkerns a);

c) eine separate äussere Schicht mit Membraneigenschaften, welche für den Wirkstoff Metoprolol im wesentlichen undurchlässig und für Körperflüssigkeit im Gastrointestinaltrakt durchlässig ist, und mit der Schicht b) fest verbunden ist und diese gleichmässig umhüllt und

d) eine Passage durch beide Schichten b) und c), welche einen Materialfluss zwischen Kern a) und der äusseren Umgebung herstellt und den Wirkstoff Metoprolol in den Gastrointestinaltrakt abgibt.

Der geformte Tablettenkern a) enthält ca. 5-20 Gew.-%, vorzugsweise 8-15 Gew.-%, Bindemittel, welche bei Anwendung der üblichen Tablettierverfahren üblich sind, ca. 0-5 Gew.-%, vorzugsweise 1-5 Gew.-%, insbesondere 2-4 Gew.-%, eines bei Tablettierverfahren üblichen Gleit- oder Schmiermittels (Lubricant), ca. 0-75 Gew.-%, vorzugsweise 0-45 Gew.-%, insbesondere 0-20 Gew.-%, eines osmotisch aktiven Treibmittels sowie ein mässig wasserlösliches, pharmazeutisch annehmbares Metoprololsalz in der gewählten Dosierung. Die angegebenen Prozentwerte beziehen sich auf das Gewicht des Kerns a).

Ein mässig wasserlösliches, pharmazeutisch annehmbares Salz von Metoprolol ist vorzugsweise das Metoprololfumarat. Dieses Salz löst sich in wässriger Phase nach Eindringen von Körperflüssigkeit aus dem Gastrointestinaltrakt durch die semipermeable Membran c) und die wasserlösliche Filmschicht b) in den Kern a) auf und bildet aufgrund seiner mässigen Wasserlöslichkeit, gegebenenfalls zusammen mit dem osmotisch aktiven Treibmittel, eine Lösung, welche auf die semipermeable Membran c) einen kontinuierlich steigenden osmotischen Druck ausübt. Da die semipermeable Membran c) starr ist, wird der sich gegen die umgebende Körperflüssigkeit bildende osmotische Druck auf die semipermeable Membran c) durch Abgabe von Flüssigkeit mit Wirkstoff und den löslichen Bestandteilen des Kerns durch die Passage d) zwischen den Schichten b) und c) ausgeglichen, welche einen Materialfluss zwischen dem Kern a) und der äusseren Umgebung ermöglicht. Der osmotische Druck auf die äussere Schicht c) wird ausserdem durch den Quelldruck des im Kern a) vorhandenen Bindemittels verstärkt bzw. überlagert. Da es sich um Gleichgewichtsvorgänge aufgrund von Diffusion und Osmose und deren Ausgleich handelt, erfolgt die Abgabe des Wirkstoffs in einer kontinuierlich und kontrollierten Weise, vorzugsweise mit gleichmässiger Geschwindigkeit. Das Metoprololsalz, insbesondere das Fumaratsalz, besitzt vorzugsweise geringe oder nur mässige Löslichkeit in wässriger Körperflüssigkeit, so dass die Abgabe des Wirkstoffs verzögert und kontinuierlich bei gleichbleibender Auflösungsgeschwindigkeit in der vom System aufgenommenen Körperflüssigkeit erfolgen kann.

Das mässig wasserlösliche Metoprololsalz besitzt vorzugsweise eine Löslichkeit in Wasser von ca. 0,1 bis 0,6 g/cm³ bei 37°C. Geeignete mässig wasserlösliche Salze, welche für pharmazeutische Zwecke verwendbar sind, sind z.B. Niederalkanoate von Mono- oder Dicarbonsäuren, z.B. das Fumarat-, Maleat- und Acetatsalz. Das Fumaratsalz ist aus den weiter oben genannten Gründen besonders bevorzugt.

Geeignete, bei Tablettierverfahren übliche Schmiermittel (Lubricants) sind z.B. Kieselgel, Stärke, Talk, Magnesiumstearat, Stearinsäure und Polyäthylenglycole mit hohem Molekulargewicht, vorzugsweise oberhalb 1000. Bevorzugt ist Magnesiumstearat.

Das osmotisch aktive Treibmittel ist vorzugsweise eine lösliche Verbindung, insbesondere ein Kohlehydrat wie Glucose, Saccharose, Dextrose, Maltose, Mannit oder Sorbitan oder ist beispielsweise ein wasserlösliches ionisches Salz, welches die anderen Bestandteile der Formulierung nicht stört, z.B. Natrium- oder Kaliumchlorid oder Harnstoff. Generell sind dissoziationsfähige Verbindungen und Salze mit physiologischer Eignung verwendbar.

Das für Tablettierverfahren übliche Bindemittel kann aus den bekannten in Pharmakopöen für diese Zwecke vorgesehenen Hilfsstoffen ausgewählt werden. Besonders bevorzugt ist Poly-N-vinylpyrrolidon mit einem durchschnittlichen Molekulargewicht von ca. 10000 bis 600000. Vorzugsweise verwendet man Povidone (U.S. Pharmakopoe), welche kommerziell unter der Bezeichnung PLASDONE (Warenzeichen der Fa. GAF) erhältlich sind. Dieses spezielle Bindemittel wird mit einem Gehalt von ca. 5-20 Gew.-%, vorzugsweise 8,5-15 Gew.-%, und besonders bevorzugt 8,5-13 Gew.-% bezogen auf das Gewicht des Kerns verwendet.

Die spezielle Eignung von Poly-N-vinylpyrrolidon als Bindemittel ergibt sich durch die Minderung des Abriebs des Metoprololsalzes im Tablettierverfahren.

Der Tablettenkern a) wird mit einer gleichmässigen separaten inneren Schicht aus ca. 0,3 bis 10 Gew.-%,

vorzugsweise 0,5 bis 2 %, aus wasserlöslichem Beschichtungsmittel bezogen auf das Gewicht des Tablettenkerns a) überzogen. Jedes pharmazeutisch verwendbare, wasserlösliche Beschichtungsmittel mit filmbildenden Eigenschaften eignet sich zur Herstellung dieser Innenschicht b). Geeignete Beschichtungsmittel sind beispielsweise Hydroxypropylmethylcellulose, Polyvinylpyrrolidon, Hydroxypropylcellulose, Hydroxyäthylcellulose, Methylcellulose, Polyäthylenglycol oder Polyoxypropylen-Polyoxyäthylen-Copolymere. Das verwendbare Polyäthylenglycol hat vorzugsweise ein durchschnittliches Molekulargewicht von ca. 400 bis 8000. Die verwendbaren Celluloseäther haben einen Viskositätsgrad von ca. 3 bis 4000 cps. Die genannten Beschichtungsmittel mit filmbildenden Eigenschaften lassen sich auch in Form von Mischungen verwenden, vorzugsweise mit einem Gehalt von ca. 50 % Hydroxypropylmethylcellulose. Besonders bevorzugt ist das Gemisch von Hydroxypropylmethylcellulose kombiniert mit geringeren Anteilen Polyäthylenglycol und/oder Polyvinylpyrrolidon. Die innere Schicht aus wasserlöslichem Filmmaterial kann ausserdem bis ca. 10 % Trennmittel enthalten, insbesondere Talk oder Titandioxid, bezogen auf das Gesamtgewicht der inneren Schicht. Das Trennmittel hat üblicherweise eine durchschnittliche Teilchengrösse von weniger als ca. 25 Mikrometer.

Die äussere Hülle c) aus einem für Körperflüssigkeit durchlässigen und für die Komponenten des wirkstoffhaltigen Kerns undurchlässigen semipermeablen Material hat film- oder schichtbildende Eigenschaften und ist sowohl gegenüber den Bestandteilen des wirkstoffhaltigen Kerns als auch der umgebenden Körperflüssigkeit inert. Das Material muss sich für die Verwendung in der Pharmazie eignen, für die umgebende Körperflüssigkeit durchlässig und für das Metoprololsalz im wesentlichen undurchlässig sein. Das Material ist in der umgebenden Körperflüssigkeit schwerlöslich oder soweit löslich, dass dieses erst gegen Ende des Durchtritts durch den Gastrointestinaltrakt gelöst ist. Für die Bildung der äusseren Hülle geeignete Materialien sind z.B. für die Herstellung von osmotischen Membranen oder Umkehrosmosemembranen bekannt und z.T. kommerziell erhältlich, z.B. Celluloseacetat, Cellulosetriacetat, Agaracetat, Amylosetriacetat, beta-Glucanacetat, beta-Glucantriacetat, Celluloseacetatäthylcarbamat, Celluloseacetatphthalat, Celluloseacetatmethylcarbamat, Celluloseacetatsuccinat, Celluloseacetatdimethylaminoacetat, Celluloseacetatäthylcarbonat, Celluloseacetatmethylsulfonat, Celluloseacetatbutylsulfonat, Celluloseacetatpropionat, Polyvinylmethyläther-Polymere, Celluloseacetatoctat, Celluloseacetatlaurat, Methylcellulose, Celluloseacetat-p-toluolsulfonat, Aethylcellulose, Triacetat von Johanniskernmehl, Celluloseacetat mit acetylierter Hydroxyäthylcellulose, Hydroxyliertes Aethylenvinylacetat, osmotische Membranen aus polymeren Epoxyden, Alkenylenoxid-Alkylglycidyl Aethern, Polyurethanen, Polyglycolsäure, und übliche Polycation-Polyanion Membranen.

Generell haben geeignete Membranen für Flüssigkeiten eine Permeabilität von ca. 0,01 bis 10 ml/cm$^2$ pro Stunde oder Tag bei Atmosphärendruck oder erhöhtem Druck gegen eine gesättigte Lösung bei ca. 30°C und sind gleichzeitig gegenüber Metoprololsalzen undurchlässig.

Besonders bevorzugte Membranmaterialien sind Polyurethane, Methylcellulose, Aethylcellulose oder Celluloseacetatbutyrat. Besonders bevorzugt ist vor allem Celluloseacetat.

Die genannten semipermeablen Membranmaterialien sind bekannt und mit ihren Eigenschaften und Verwendungsmöglichkeiten in der U.S. Patentschrift 3,916,899 beschrieben.

Die Menge des im Kern a) vorhandenen Metoprololsalzes ist variierbar und entspricht der für den Wirkstoff Metoprolol vorgeschriebenen zulässigen Höchst- und Mindestdosierung. Bezogen auf die Base können ca. 25 bis 500 mg in der Einheitsformulierung vorhanden sein. Bevorzugt enthält der Kern 30-400 mg, insbesondere 100 bis 400 mg oder 200 bis 400 mg, und vor allem 250-400 mg Metoprolol in Form eines pharmazeutisch verwendbaren Salzes.

Der Kern a) liegt vorzugsweise als tablettenförmige Formulierung vor, welche mit der separaten inneren Schicht b) aus wasserlöslichem Filmmaterial und dem Membranmaterial der Hülle c) überzogen ist. Die Herstellung des Kerns kann beispielsweise erfolgen, indem das mässig wasserlösliche Metoprololsalz mit dem Poly-N-vinylpyrrolidon direkt trocken verpresst oder als Granulat verpresst wird. Die Herstellung des letzteren erfolgt z.B. durch Vermischen und Vortrocknen einer wässrig äthanolischen Suspension der Komponenten und Zerkleinerung und Trocknen des Rückstandes oder durch Auflösen oder Suspendieren des Metoprololsalzes in einer wässrigen äthanolischen Lösung von Poly-N-vinylpyrrolidon, Vortrocknen, Zerkleinern, Entfernen des restlichen Lösungsmittels, Mahlen des Granulats auf einheitliche Kerngrösse und Verpressen des Granulats in Gegenwart eines Gleitmittels wie Magnesiumstearat zu tablettenförmigen Kernen.

Durch Verwendung von Poly-N-vinylpyrrolidon als Hilfsstoff in der angegebenen Menge von ca. 7,5-15 %, vorzugsweise 8,5-13 %, lassen sich tablettenförmige Kerne geeigneter Härte herstellen, deren Härtegrade ca. 8-25 SCU (Strong Cobb Units, Strong Cobb hardness tester, pg. 1608 Remington's Pharmaceutical Sciences, March 1985), insbesondere 8-20 SCU, und Bruchfestigkeit, gemessen in prozentualen Anteilen an zerbrochenen Tablettenkernen, nicht grösser als 2,5 %, insbesondere nicht grösser als 1 % ist.

Die innere Schicht b) kann auf den den Wirkstoff enthaltenden Kern a) durch Aufgiessen, Formen, Sprühen oder Eintauchen des Kerns in eine Lösung des wasserlöslichen Beschichtungsmittels, z.B. Hydroxypropylmethylcellulose, aufgetragen werden. Auf diesen beschichteten Kern kann wiederum durch Aufgiessen, Formen, Sprühen oder Eintauchen das die semipermeable Hülle c) bildende Material aufgebracht werden. Ein anderes Verfahren, das zum Aufbringen der beiden Schichten angewandt werden kann, besteht im Luftverwirbeln (air suspension procedure). Dieses Verfahren besteht darin, dass man die Massen (Tabletten bzw. Tablettenkerne) in einem Luftstrom und einem die jeweilige Schicht bildenden Mittel suspendiert und stürzt, bis die Schichten nacheinander den Kern umgeben und überziehen. Das Luftverwirbelungs-Verfahren ist in der U.S. Patentschrift 2 799 241 sowie im J.Am. Pharm. Assoc., Bd. 48, S. 451 - 459, 1979, und im Band 49, S. 82 bis 84, 1980, beschrieben. Andere bevorzugte Standardverfahren sind beispielsweise das Kessellackierungsverfahren (spray pan), welches in Remington's Pharmaceutical Sciences, 14th Edition, auf den Seiten 1686 - 1687 beschrieben ist. Bei Verwendung von Celluloseacetat als membranbildendem semipermeablem Material hat die Hülle a) einen Anteil von ca. 4-30 % (Gew.), vorzugsweise 10-20 %, am Gesamtgewicht der Formulierung.

Der Begriff "Passage durch die Hülle a) für den Transport der im Kern enthaltenen Bestandteile in die umgebende wässrige Körperflüssigkeit" umfasst Vorrichtungen sowie Methoden, die geeignet sind, die Wirkstoffzubereitung aus dem Kern des therapeutischen Systems freizusetzen. Der Begriff umfasst Durchgänge, Oeffnungen, Bohrungen, Löcher u.a. durch die als semipermeable Membran wirkende Hülle a), welche zwischen der Oberfläche der Hülle und dem Kern eine Verbindung herstellen. Die Passage kann durch mechanisches Bohren oder Laserbohren oder durch Zersetzen eines abbaubaren Bestandteils, z.B. eines Gelatinestopfens, unter Bildung einer Passage in der Hülle des therapeutischen Systems hergestellt werden. Bei einer Ausführungsform kann sich die Passage als Reaktion auf den hydrostatischen Druck bilden, welcher auf das therapeutische System einwirkt. Bei einer anderen Ausführungsform können zwei oder mehrere Durchgänge hergestellt werden, die sich an einer beliebigen Stelle des Systems befinden. Die Passage kann auch durch mechanisches Aufbrechen der Schichten während der Anwendung des Systems entstehen. Die Passage hat einen minimalen Durchmesser, der von der Korngrösse der Wirkstoffkristalle abhängig ist. Der Durchmesser der Passage muss grösser als die durchschnittliche Länge der Wirkstoffkristalle sein. Der maximale Durchmesser ist ebenfalls annähernd festgelegt. Dieser darf nur so gross sein, dass das Eindringen von wässriger Körperflüssigkeit durch Konvektion in das therapeutische System vermieden wird. Eine genaue Beschreibung der Herstellung der Passage und der maximalen und minimalen Dimensionen davon ist in den U.S. Patentschriften 3 485 770 und 3 916 899 und den dazugehörigen Zeichnungen enthalten. Die Grösse der Oeffnung beträgt in einer bevorzugten Ausführungsform ca. 0,1-0,8 mm.

Das erfindungsgemässe therapeutische System kann unterschiedlich geformt sein und beispielsweise rund, oval, oblong, zylindrisch u.a. sein sowie verschiedene Grössen in Abhängigkeit von der Füllmenge haben. Das therapeutische System kann ausserdem transparent, farblos oder gefärbt und gegebenenfalls beschriftet sein, um diesem Produkt ein individuelles Aussehen bzw. sofortige Erkennbarkeit zu verleihen.

Das erfindungsgemässe orale osmotische System für die Verabreichung eines Metoprololsalzes eignet sich für die Verwendung in Heilverfahren zur Behandlung von Krankheiten oder Zuständen, welche für die Verwendung von Beta$_1$-Adrenalinrezeptorblockern (Betablocker) indiziert sind. Bevorzugt sind die Indikationen, welche für Metoprolol selbst und seine pharmazeutisch annehmbaren Salze bekannt sind, insbesondere Bluthochdruck, Angina Pectoris, Herzrhythmusstörungen oder bei der Nachsorge von Patienten mit Myokardinfarkt.

Bei Verwendung von Metoprololtartrat in konventionellen Formulierungen wie Tabletten ist das Abgabeverhalten der Darreichungsform durch sofortige Abgabe des Wirkstoffs gekennzeichnet. Es fehlt ein geschwindigkeitskontrolliertes, kontinuierliches Abgabeverhalten. Bei erforderlicher Mehrfachdosierung erzeugen solche Formulierungen grosse Schwankungen des Blutplasmaspiegels zwischen hohen und niedrigen Konzentrationen des Wirkstoffs unter Verminderung der Effektivität der Betablockade. Häufige Zufuhr des Wirkstoffs kann zwar das Absinken des Blutplasmaspiegels unter das therapeutische Niveau verhindern, stellt aber insgesamt eine Belastung des Organismus dar. Ausserdem besteht das Risiko der unbeabsichtigten nicht regelmässigen Einnahme. Für diesen Wirkstoff sind verschiedene Verabreichungsintervalle vorgesehen, deren genaue Einhaltung überwacht werden muss, z.B. einmal täglich bei Patienten zur Verhinderung des Bluthochdrucks, zweimal täglich zur Verminderung des Reinfarktrisikos und bei Anzeichen von Angina Pectoris sowie dreimal täglich zur Verhinderung von Herzrhythmusstörungen.

Das erfindungsgemässe orale osmotische System erlaubt eine einmal tägliche Verabreichung für alle genannten Indikationen, wobei von der Einheitsdosis von ca. 50 bis 500 mg Metoprolol ca. 50 bis 90 % der Gesamtmenge mit der kontinuierlichen Abgabegeschwindigkeit von ca. 5 bis 12 % pro Stunde abgegeben werden.

Ebenfalls Gegenstand der vorliegenden Erfindung ist ein humantherapeutisches Verfahren zur Behandlung von Krankheiten und/oder Zuständen, welche für die Verwendung von Beta$_1$-Adrenalinrezeptorblockern (Betablocker) indiziert sind, welches dadurch gekennzeichnet ist, dass dem Patienten das obige osmotische System mit einer effektiven Einheitsdosis des Wirkstoffs oral verabreicht wird.

Beispiel 1

3800 g Metoprololfumarat und 190 g Hydroxypropylmethylcellulose werden jeweils durch ein Sieb mit 0,55 und 0,99 mm (30 und 16 Mesh (U.S. Pharmacopoe)) Maschenweite gegeben. Man vermischt das Siebgut miteinander und granuliert dieses mit einer 10 %-igen (w/v) Lösung Polyvinylpyrrolidon in einer 70:30-Mischung Aethanol und Wasser. Das Granulat wird 16 Stunden lang bei 40°C getrocknet, gemahlen, anschliessend mit 120 g Magnesiumstearat vermischt und zu Tablettenkernen komprimiert.

56,0 g Hydroxypropylmethylcellulose werden in 750 ml einer Methanol/Methylenchlorid-Mischung gelöst. Mit dieser Lösung werden die komprimierten Tablettenkerne unter Anwendung des Luftwirbelverfahrens einheitlich beschichtet. 24 g Hydroxypropylmethylcellulose, 24 g Polyäthylenglycol und 252 g Celluloseacetat werden in 5000 ml einer Methanol/Methylenchlorid-Mischung gelöst und mit dieser Lösung die mit Hydroxypropylmethylcellulose beschichteten Tablettenkerne unter Anwendung eines Luftwirbelverfahrens einheitlich beschichtet. In die beschichteten Tabletten wird eine ca. 0,34 mm starke Durchbohrung angebracht, welche durch beide Aussenschichten reicht.

Beispiel 2

Analog dem Verfahren gemäss Beispiel 1 werden folgende orale osmotische Systeme hergestellt:

| Wirkstoff | [g] | Bindemittel | [g] | Granulier-mittel | [g] | Zwischen-schicht | [g] |
|---|---|---|---|---|---|---|---|
| Metoprolol-fumarat | 3800 | | | | | | |
| Metoprolol-tartrat | 4000 | | | | | | |
| | | HPC | 190 | | | | |
| | | | | HPMC | 190 | | |
| | | HPMC | 380 | | | | |
| | | | | HPC | 190 | | |
| | | HEC | 190 | | | | |
| | | PVP | 400 | | | HPMC | 30 |
| | | | | | | HPMC PVP | 30 28 |

HPC: Hydroxypropylcellulose

HEC: Hydroxyäthylcellulose

PVP: Polyvinylpyrrolidon

HPMC: Hydroxypropymethylcellulose

## Patentansprüche

### Patentansprüche für folgende Verstragsstaaten : AT, BE, CH, DE, GB, IT, LI, LU, NL, SE

1. Orales osmotisches System für die kontrollierte und kontinuierliche Applikation von Metoprolol in einem verlängerten Zeitintervall enthaltend folgende Bestandteile:

a) einen geformten Tablettenkern mit einem osmotisch aktiven Gehalt eines pharmazeutisch annehmbaren Metoprololsalzes kombiniert mit einem pharmazeutisch annehmbaren und für Tablettierverfahren üblichen Bindemittel und gegebenenfalls einem osmotisch aktiven Treibmittel und/oder weiteren pharmazeutischen Hilfsstoffen;

b) eine gleichmässige separate, innere Schicht aus ca. 0,3 % - 10 % (Gew.) wasserlöslichem Beschichtungsmittel bezogen auf das Gewicht des Tablettenkerns a);

c) eine separate äussere Schicht mit Membraneigenschaften, welche für den Wirkstoff Metoprolol im wesentlichen undurchlässig und für Körperflüssigkeit im Gastrointestinaltrakt durchlässig ist, und mit der Schicht b) fest verbunden ist und diese gleichmässig umhüllt und

d) eine Passage durch beide Schichten b) und c), welche einen Materialfluss zwischen Kern a) und der äusseren Umgebung herstellt und den Wirkstoff Metoprolol in den Gastrointestinaltrakt abgibt.

2. Orales osmotisches System gemäss Anspruch 1, dadurch gekennzeichnet, dass die gleichmässige separate innere Schicht aus Hydroxypropylmethylcellulose oder Polyvinylpyrrolidon oder Mischungen davon besteht.

3. Orales osmotisches System gemäss Anspruch 1, dadurch gekennzeichnet, dass die gleichmässige separate innere Schicht aus Hydroxypropylmethylcellulose, Polyvinylpyrrolidon oder Mischungen mit bis 50 % Polyäthylenglycol besteht.

4. Verfahren zur Herstellung eines oralen osmotischen Systems gemäss Anspruch 1, dadurch gekennzeichnete dass man den Tablettenkern a) durch Komprimieren eines Granulats herstellt, diesen mit dem wasserlöslichen Beschichtungsmittel der Schicht b) versieht, darauf die separate äussere Schicht c) mit Membraneigenschaften anbringt und die Passage d) durch die Schichten b) und c) herstellt.

### Patentansprüche für folgende Vertragsstaaten: ES, GR

1. Verfahren zur Herstellung eines oralen osmotischen Systems für die kontrollierte und kontinuierliche Applikation von Metoprolol in einem verlängerten Zeitintervall, welches folgende Bestandteile enthält:

a) einen geformten Tablettenkern mit einem osmotisch aktiven Gehalt eines pharmazeutisch annehmbaren Metoprololsalzes kombiniert mit einem pharmazeutisch annehmbaren und für Tablettierverfahren üblichen Bindemittel und gegebenenfalls einem osmotisch aktiven Treibmittel und/oder weiteren pharmazeutischen Hilfsstoffen;

b) eine gleichmässige separate, innere Schicht aus ca. 0,3 % - 10 % (Gew.) wasserlöslichem Beschichtungsmittel bezogen auf das Gewicht des Tablettenkerns a);

c) eine separate äussere Schicht mit Membraneigenschaften, welche für den Wirkstoff Metoprolol im wesentlichen undurchlässig und für Körperflüssigkeit im Gastrointestinaltrakt durchlässig ist, und mit der Schicht b) fest verbunden ist und diese gleichmässig umhüllt und

d) eine Passage durch beide Schichten b) und c), welche einen Materialfluss zwischen Kern a) und der äusseren Umgebung herstellt und den Wirkstoff Metoprolol in den Gastrointestinaltrakt abgibt, dadurch gekennzeichnet, dass man den Tablettenkern a) durch Komprimieren eines Granulats herstellt, diesen mit dem wasserlöslichen Beschichtungsmittel der Schicht b) versieht, darauf die separate äussere Schicht c) mit Membraneigenschaften anbringt und die Passage d) durch die Schichten b) und c) herstellt.

2. Verfahren gemäss Anspruch 1, dadurch gekennzeichnet, dass die gleichmässige separate innere Schicht aus Hydroxypropylmethylcellulose oder Polyvinylpyrrolidon oder Mischungen davon besteht.

3. Verfahren gemäss Anspruch 1, dadurch gekennzeichnet, dass die gleichmässige separate innere Schicht aus Hydroxypropylmethylcellulose, Polyvinylpyrrolidon oder Mischungen mit bis 50 % Polyäthylenglycol besteht.

## Claims

### Claims for the following Contracting States : AT, BE, CH, DE, FR, GB, IT, LI, LU, NL, SE

1. An oral osmotic system for the controlled and continuous administration of metoprolol over a prolonged period of time, comprising the following constituents:

a) a shaped tablet core comprising an osmotically active composition comprising a pharmaceutically acceptable metoprolol salt in combination with a pharmaceutically acceptable binder customary in tabletting processes and, optionally, an osmotically active propellant and/or other pharmaceutical excipients;

b) a uniform, discrete inner layer comprising approximately from 0.3 % to 10 % (by weight) water-soluble coating composition, based on the weight of the tablet core a);

c) a discrete outer layer having membrane properties which is substantially impermeable to the active ingredient metoprolol and is permeable to body fluid in the gastrointestinal tract, and which is firmly bonded to the layer b) and coats it evenly; and

d) a passageway through the two layers b) and c) which produces a flow of material between the core a) and the external environment and releases the active ingredient metoprolol into the gastrointestinal tract.

2. An oral osmotic system according to claim 1, wherein the uniform discrete inner layer consists of hydroxypropylmethylcellulose or polyvinylpyrrolidone or mixtures thereof.

3. An oral osmotic system according to claim 1, wherein the uniform discrete inner layer consists of hydroxypropylmethylcellulose, polyvinylpyrrolidone or mixtures comprising up to 50 % polyethylene glycol.

4. A process for the preparation of an oral osmotic system according to claim 1, which comprises preparing the tablet core a) by compressing granules, providing the tablet core a) with the water-soluble coating composition of layer b), applying thereto the discrete outer layer c) having membrane properties, and forming the passageway d) through the layers b) and c).

## Claims for the following Contractrin States : ES, GR

1. A process for the preparation of an oral osmotic system for the controlled and continuous administration of metoprolol over a prolonged period of time, comprising the following constituents:

a) a shaped tablet core comprising an osmotically active composition comprising a pharmaceutically acceptable metoprolol salt in combination with a pharmaceutically acceptable binder customary in tabletting processes and, optionally, an osmotically active propellant and/or other pharmaceutical excipients;

b) a uniform, discrete inner layer comprising approximately from 0.3 % to 10 % (by weight) water-soluble coating composition, based on the weight of the tablet core a);

c) a discrete outer layer having membrane properties which is substantially impermeable to the active ingredient metoprolol and is permeable to body fluid in the gastrointestinal tract, and which is firmly bonded to the layer b) and coats it evenly; and

d) a passageway through the two layers b) and c) which produces a flow of material between the core a) and the external environment and releases the active ingredient metoprolol into the gastrointestinal tract, which process comprises preparing the tablet core a) by compressing granules, providing the tablet core a) with the water-soluble coating composition of layer b), applying thereto the discrete outer layer c) having membrane properties, and forming the passageway d) through the layers b) and c).

2. A process according to claim 1, wherein the uniform discrete inner layer consists of hydroxypropylmethylcellulose or polyvinylpyrrolidone or mixtures thereof.

3. A process according to claim 1, wherein the uniform discrete inner layer consists of hydroxypropylmethylcellulose, polyvinylpyrrolidone or mixtures comprising up to 50 % polyethylene glycol.

## Revendications

## Revendications pour les Etats contractants suivants : AT, BE, CH, DE, FR, GB, IT, LI, LU, NL, SE

1. Système osmotique oral pour l'administration contrôlée et continue du Metoprolol dans une durée prolongée, contenant les constituants suivants :

a) un noyau de comprimé moulé contenant, à l'état osmotique actif, un sel de Metoprolol acceptable pour l'usage pharmaceutique combiné avec un liant acceptable pour l'usage pharmaceutique et usuel pour les mises en comprimés, et le cas échéant un véhicule osmotique actif et/ou d'autres produits auxiliaires pharmaceutiques;

b) une couche intérieure séparée uniforme consistant en environ 0,3 à 10 % en poids d'un produit de revêtement soluble dans l'eau, par rapport au poids du noyau de comprimé a);

c) une couche extérieure séparée ayant des propriétés de membrane, essentiellement imperméable pour la substance active Metoprolol mais perméable dans la voie gastro-intestinale pour les liquides physiologiques, solidement associée à la couche b) et enrobant uniformément cette dernière, et

d) un passage au travers des deux couches b) et c), établissant un flux de matière entre le noyau a) et l'environnement extérieur, et libérant la substance active Metoprolol dans la voie gastro-intestinale.

2. Système osmotique oral selon revendication 1, caractérisé en ce que la couche intérieure séparée, uniforme, consiste en hydroxypropylméthylcellulose ou polyvinylpyrrolidone ou mélanges de ces substances.

3. Système osmotique oral selon revendication 1, caractérisé en ce que la couche intérieure, séparée, uniforme, consiste en hydroxypropylméthylcellulose, polyvinylpyrrolidone ou mélanges de ces substances avec des proportions allant jusqu'à 50 % d'un polyéthylène-glycol.

4. Procédé de préparation d'un système osmotique oral selon revendication 1, caractérisé en ce que l'on prépare le noyau de comprimé a) par compression de granulés, on applique sur le noyau de comprimé le produit de revêtement hydrosoluble de la couche b) puis, sur celui-ci, la couche extérieure séparée c) ayant des propriétés de membrane, et on pratique le passage d) au travers des couches b) et c).

**Revendications pour les Etats contractants suivants : ES, GR**

1. Procédé de préparation d'un système osmotique oral pour l'administration contrôlée et continue du Metoprolol dans des durées prolongées, contenant les constituants suivants :

a) un noyau de comprimé moulé contenant, à l'état osmotique actif, un sel de Metoprolol acceptable pour l'usage pharmaceutique combiné avec un liant acceptable pour l'usage pharmaceutique et usuel pour les mises en comprimés et le cas échéant un véhicule osmotique actif et/ou d'autres produits auxiliaires pharmaceutiques;

b) une couche intérieure, séparée, uniforme consistant en environ 0,3 à 10 % en poids d'un produit de revêtement hydrosoluble par rapport au poids du noyau de comprimé a);

c) une couche extérieure séparée ayant des propriétés de membrane, essentiellement imperméable pour la substance active Metoprolol, mais perméable pour le liquide physiologique dans la voie gastro-intestinale, solidement associée avec la couche b) et enrobant uniformément cette dernière, et

d) un passage au travers des deux couches b) et c), établissant un flux de matière entre le noyau a) et l'environnement extérieur et libérant la substance active Metoprolol dans la voie gastro-intestinale, caractérisé en ce que l'on prépare le noyau de comprimé a) par compression de granulés, on applique sur ce noyau le produit de revêtement hydrosoluble de la couche b) puis, sur celui-ci, la couche extérieure séparée c) ayant des propriétés de membrane, et on pratique le passage d) au travers des couches b) et c).

2. Procédé selon la revendication 1, caractérisé en ce que la couche uniforme intérieure, séparée, consiste en hydroxypropylméthylcellulose ou polyvinylpyrrolidone ou mélanges de ces substances.

3. Procédé selon la revendication 1, caractérisé en ce que la couche uniforme, intérieure, séparée consiste en hydroxypropylméthylcellulose, polyvinylpyrrolidone ou mélanges de ces substances avec une proportion allant jusqu'à 50 % de polyéthylèneglycol.